# EUROPEAN PATENT APPLICATION

(11) **EP 4 592 677 A1**
(43) Date of publication of application: **30.07.2025**
(21) Application number: 23868116.7
(22) Date of filing: 13.09.2023
(51) Int. Cl.: G01N 33/53

(54) **METHOD FOR ASSISTING IN DETERMINING TREATMENT PLAN FOR DOG HAVING SKIN CONDITION**

(30) Priority: 20.09.2022 JP 2022148724
(71) Applicant: Nippon Zenyaku Kogyo Co., Ltd., Koriyama-shi, Fukushima 963-0196 (JP)
(72) Inventor: TSUKUI, Toshihiro, Koriyama-shi, Fukushima 963-0196 (JP); OGINO, Shoji, Koriyama-shi, Fukushima 963-0196 (JP); FAVROT, Claude, 68100 Mulhouse (FR)
(74) Representative: Mewburn Ellis LLP
(86) International application number: PCT/JP2023/033430
(87) International publication number: WO 2024/062993

(57) **Abstract**

Provided is a method for assisting in determining a treatment strategy for a dog with an atopic dermatitis-like skin symptom by using an objective indicator based on a blood test. Provided is a method comprising the following steps: step a) of measuring a level of TARC comprised in a blood sample collected from the subject; and step b) of determining that treatment for atopic dermatitis is useful when the level of TARC is equal to or greater than a threshold value, and determining that treatment for a different disease is useful when the level of TARC is less than the threshold value.

## Description

### Technical Field

The present invention relates to a method for assisting in determining a treatment strategy for a dog with a canine atopic dermatitis-like skin symptom, and a reagent for use in the same.

### Background Art

Atopic dermatitis is a disease having long-term recurring chronic eczematous skin lesions, often with an atopic predisposition. Meanwhile, hematological findings recognized often include abnormalities such as increased eosinophil counts in the peripheral blood and high serum IgE levels. Immunological and non-immunological abnormalities have been reported as major factors in atopic dermatitis. Recently, atopic dermatitis has been increasing not only in humans but also in pet animals such as dogs, and it is desirable to establish detection and treatment methods for this disease.

It has been reported that blood TARC levels are high in human patients with atopic dermatitis and show a correlation with the disease state (see Non Patent Literatures 1 and 2). On the other hand, it has been reported that TARC is also expressed in healthy human tissues, and the usefulness of TARC as, for example, a diagnostic marker or a therapeutic target has not yet been fully investigated.

The relationship between atopic dermatitis and TARC has been extensively studied in mouse models and human. However, the pathogenesis of atopic dermatitis is not always consistent among animals, and it has been desirable to establish a canine-specific disease marker and a diagnostic method. Patent Literature 1 reports an antibody that specifically recognizes canine TARC. Patent Literature 2 reports a method for diagnosing canine atopic dermatitis by using TARC in canine serum as a biomarker.

In addition to atopic dermatitis, canine skin diseases are known to include scabies, acne mites, infections, and food allergy. Some of these diseases exhibit skin findings similar to those of atopic dermatitis and are difficult to differentiate. Unfortunately, effective treatments for these diseases differ from disease to disease. Therefore, it is desirable to accurately differentiate these diseases in their early stages for early cure.

### Citation List

### Patent Literature

Patent Literature 1: International Publication No. WO 2017/065203
Patent Literature 2: JP Patent Publication (Kokai) No. 2019-191007

### Non Patent Literature

Non Patent Literature 1: Takao. Fujisawa et al, The Japanese Journal of Pediatric Allergy and Clinical Immunology, Vol. 19, No. 5, p. 744-757 (2005)
Non Patent Literature 2: Kunihiko Tamaki et al, The Japanese Journal of Dermatology, Vol. 116, No. 1, p. 27-39 (2006)

### Summary of Invention

### Technical Problem

The purpose of the present invention is to provide a method for assisting in determining a treatment strategy for a dog with an atopic dermatitis-like skin symptom by using an objective indicator based on a blood test and a reagent and a kit for use in the same.

### Solution to Problem

The present invention is as follows:
[1] A method for assisting in determining a treatment strategy for a canine subject with an atopic dermatitis-like skin symptom, comprising the steps of:
   a) measuring a level of TARC comprised in a blood sample collected from the subject; and
   b) determining that treatment for atopic dermatitis is useful when the level of TARC is equal to or greater than a threshold value, and determining that treatment for a different disease is useful when the TARC level is less than the threshold value.
[2] The method according to [1], wherein in the step b), the different disease is at least one disease selected from the group consisting of scabies, acne mites, flea parasites, tick parasites, lice parasites, bacterial infections, fungal infections (including yeast infections), immune-mediated diseases (including psoriasis and food allergy), stress-induced scratching, mastocytoma, and cutaneous lymphomas.
[3] The method according to [2], wherein the different disease is scabies.
[4] The method according to any of [1] to [3], wherein in the step a), the level of TARC is measured using an antibody that specifically binds to canine TARC.
[5] A reagent for assisting in determining a treatment strategy for a canine subject with an atopic dermatitis-like skin symptom, wherein
   the reagent has a function to measure a level of TARC comprised in a blood sample collected from the subject, and
   the treatment strategy determination comprises determining that treatment for atopic dermatitis is useful when the level of TARC is equal to or greater than a threshold value, and determining that treatment for a different disease is useful when the level of TARC is less than the threshold value.
[6] The reagent according to [5], wherein the different disease is at least one disease selected from the group consisting of scabies, acne mites, flea parasites, tick parasites, lice parasites, bacterial infections, fungal infections (including yeast infections), immune-mediated diseases (including psoriasis and food allergy), stress-induced scratching, mastocytoma, and cutaneous lymphomas.
[7] The reagent according to [6], wherein the different disease is scabies.
[8] The reagent according to any of [5] to [7], comprising an antibody that specifically binds to canine TARC.
[9] A method for assisting in a differential diagnosis of atopic dermatitis in a canine subject, comprising the steps of:
   a) measuring a level of TARC comprised in a blood sample collected from the subject; and
   b) determining that the subject suffers from atopic dermatitis when the level of TARC is equal to or greater than a threshold value, and determining that the subject suffers from a different disease when the level of TARC is less than the threshold value.
[10] A reagent for assisting in a differential diagnosis of atopic dermatitis in a canine subject,
   the reagent comprising a function to measure a level of TARC comprised in a blood sample collected from the subject, wherein
   the differential diagnosis comprises determining that the subject suffers from atopic dermatitis when the level of TARC is equal to or greater than a threshold value, and determining that the subject suffers from a different disease when the level of TARC is less than the threshold value.

The present specification encompasses the contents disclosed in Japanese Patent Application No. 2022-148724 from which the present application claims priority.

### Advantageous Effects of Invention

The present invention can provide a method for assisting in determining a treatment strategy for a dog with an atopic dermatitis-like skin symptom by using an objective indicator based on a blood test and a reagent and a kit for use in the same.

### Description of Embodiments

### 1. First Embodiment - Method for assisting in determining treatment strategy

The first embodiment of the present invention is a method for assisting in determining a treatment strategy for a canine subject with an atopic dermatitis-like skin symptom, comprising the steps a) and b) below:
step a) of measuring a level of TARC comprised in a blood sample collected from the subject; and
step b) of determining that treatment for atopic dermatitis is useful when the level of TARC is equal to or greater than a threshold value, and determining that treatment for a different disease is useful when the level of TARC is less than the threshold value.

### 1-1 Definition and Overview

In the method of this embodiment, the subject for which a treatment strategy is determined is a dog. As used herein, the term "dog" or "canine" refers to all those belonging to *Canis lupus familiaris* and should include any breed of dog.

TARC (Thymus Activation-Regulated Chemokine, also called "CCL17") is a chemokine that binds to CC chemokine receptor 4 (CCR4) and induces leukocyte migration and invasion *in vivo.* The TARC to be measured in this embodiment is TARC in canine blood samples. As used herein, TARC refers to TARC of canine origin. Specifically, it refers to a protein that comprises the amino acid sequence represented by SEQ ID NO: 1 or an amino acid sequence having 90% or more, preferably 91% or more, 92% or more, 93% or more, 94% or more, 95% or more, 96% or more, 97% or more, 98% or more, or 99% or more sequence identity with the amino acid sequence represented by SEQ ID NO: 1 and that has binding activity to CCR4.

Known clinical symptoms of atopic dermatitis include skin erythema, lichenification, inflammation, pigmentation, skin symptoms such as scratch marks and alopecia, and itching. Many different diseases with similar clinical symptoms are known. Examples of the different diseases include, but are not particularly limited to, parasites-related diseases (e.g., scabies, acne mites, flea parasites, tick parasites, lice parasites), infections such as bacterial infections (e.g., staphylococcal infections) and fungal infections (including yeast (Malassezia) infections), immune-mediated diseases (including psoriasis and food allergy), stress-induced scratching, and tumors such as mastocytoma and cutaneous lymphomas. Among these diseases, scabies is a disease that is particularly difficult to distinguish from atopic dermatitis by appearance, and the like.

In dogs with atopic dermatitis-like skin symptoms, atopic dermatitis has been diagnosed by the following protocol. First, an antibiotic or the like is administered to rule out parasitic diseases and infections. Next, food allergy is ruled out by IgE test and food load test. Further, the presence or absence of upregulation of IgE against environmental allergens is checked to rule out other immune-mediated diseases. In this way, the diagnosis of a dog with atopic dermatitis involves a process of treatment with drugs for the different diseases and confirmation of their efficacy. Thus, the problems include the time required to identify the disease, the cost of ineffective treatment, and the risk of side effects.

Parasites-related diseases and infections can be differentiated if parasitic/infecting organisms can be collected and identified. However, it is not easy to collect and identify parasitic/infecting organisms. In some individuals, the collection and identification are impossible in some cases. The method did not always allow for a reliable differential diagnosis.

Many of the different diseases mentioned above are treated differently from atopic dermatitis. Examples of the known treatment of canine atopic dermatitis include: prednisolone (Asahi Kasei Pharma, NIPRO, Towa Pharmaceutical, KYORIN Remedio, Kyorin Pharmaceutical, KOA ISEI, NIHON GENERIC, Teva Takeda Pharma, Takeda pharmaceutical Company, Mylan Seiyaku, Pfizer, Yoshindo, Shionogi); antihistamines (Nichi-Iko Pharmaceutical, TAKATA Pharmaceutical, ISEI, Sawai Pharmaceutical, Towa Pharmaceutical, Tsuruhara pharmaceutical, Taiyo Pharmaceutical); cyclosporine (Elanco, Virbac, ASKA Pharmaceutical, VMDP); desensitization therapy (Nippon Zenyaku Kogyo); administration of interferon-y (Toray), oclacitinib (Zoetis), anti-IL-31 antibody drug (Zoetis), and the like; and moisturizing shampoo therapy.

On the other hand, examples of the treatment considered effective for the parasites-related diseases such as scabies, acne mites, flea parasites, tick parasites, and lice parasites include the treatments with ivermectin (product name: CARDOMEC(R) (Boehringer Ingelheim Animal Health)), Panamectin(R) (Meiji Seika Pharma), Azavasuca(R) (Nisshin Pharmaceutical), HEARTMECTIN(R) (Aska Animal Health), STROMECTOL(R) (Maruho, MSD), and the like), imidacloprid (product name: Advocate(TM) (Bayer)), selamectin (product name: Revolution(R) (Zoetis)), saloraner (product name: Simparica(R) (Zoetis)), and fipronil (product names: Frontline(R) and Frontline(R) Plus (Boehringer Ingelheim Animal Health)).

Examples of the treatment considered effective for bacterial and yeast (Malassezia) infections include the treatments with fusidic acid and betamethasone (product name: IsaDerm(TM) (Dechra)), gentamicin sulfate (product name: GENTACALM(TM) (Dechra Veterinary Products)), cephalexin (product name: Cefaclear(R) (Riken Vets Animal Pharma)), cefaseptin (Vetoquinol(R) (Nippon Zenyaku Kogyo), Therios(R) cj (CEVA), Therios(R) (DS Pharma Animal Health), Rilexipet(R) (Virbac), and the like), gentamicin sulfate (product name: Tiacil(TM) (Virbac), Easotic(TM) (Virbac), Otomax(R) (MSD), GENTACALM(TM) (Dechra), and the like), amoxicillin and clavulanate (product name: CLAVAMOX(R) (Zoetis)), and ciprofloxacin (Teva). Further examples of the known treatment effective for yeast (Malassezia) infections include uses of miconazole nitrate (product name: MALASEB(R) Shampoo (DermaCare)), Surolan Ear Drops(TM) (Elanco), Easotic(TM) (Virbac), and the like), Polymyxin B sulfate (product name: Surolan Ear Drops(TM) (Elanco)), hydrocortisone aceponate (product name: Surolan Ear Drops(TM) (Elanco)), and gentamicin sulfate (product name: Surolan Ear Drops(TM) (Elanco)). Examples of the known treatment effective for fungal infections include uses of itraconazole (Nippon Chemiphar, Sawai Pharmaceutical, Nichi-Iko Pharmaceutical, Meiji Seika Pharma, Kobayashi Kako, KAKEN Pharmaceutical), and ITRIZOLE (Janssen Pharmaceutical).

Examples of the known treatment of immune-mediated diseases (psoriasis, food allergy) include prednisolone (Asahi Kasei Pharma, NIPRO, Towa Pharmaceutical, KYORIN Remedio, Kyorin Pharmaceutical, KOA ISEI, NIHON GENERIC, Teva Takeda Pharma, Takeda Pharmaceuticals, Mylan Seiyaku, Pfizer, Yoshindo, Shionogi). Among immune-mediated diseases, food allergy requires the identification of allergens and their elimination from the diet.

Examples of the known treatment of stress-induced scratching include uses of tryptophan (product name: Vet's Best(R) (Hero Pet brands)), valerian (product name: Vet's Best(R) (Hero Pet brands)), dexmedetomidine (product name: Sileo(TM) (Zoetis)), clomipramine hydrochloride (product name: Clomicalm(R) (Elanco)), and clomipramine hydrochloride (product name: CLOFRANIL(TM) (Sun Pharma Laboratories)).

Examples of the known treatment of mastocytoma include uses of toceranib phosphate (product name: Palladia(R) (Zoetis)), imatinib (product name: Glivec(R) (Novartis Pharma)), and imatinib (DAIICHI SANKYO ESPHA, DAIICHI SANKYO, Elmed, Nichi-Iko Pharmaceutical, NIHON GENERIC, KYOSOMIRAI PHARMA, Kobayashi Kako, Nippon Kayaku, Ohara Pharmaceutical Industry, Nippon Chemiphar, Sawai Pharmaceutical, Takeda Pharmaceutical Company, Teva Takeda Pharma, Towa Pharmaceutical, Kyowa CritiCare, NIPRO, Mylan Seiyaku, Pfizer, Yakult, Takata Pharmaceutical, Meiji Seika Pharma, Tatsumi Kagaku). Examples of the known treatment of cutaneous lymphoma include use of Lomustine (Mediclone).

The method of this embodiment makes it possible to determine at an early stage whether or not treatment indicated for atopic dermatitis is effective for a canine subject with an atopic dermatitis-like symptom, without going through the process of confirmation of the efficacies of therapeutic agents for the different diseases, and the like. This can reduce the duration, cost, and risk of side effects of treatment.

### 1-2 Step a)

Step a) of the method of this embodiment is a step of measuring a level of TARC comprised in a blood sample collected from a canine subject.

It is possible to use, as the blood sample, serum, plasma, or whole blood, and serum is particularly suitable. The method for measuring the level of TARC in the blood sample is not particularly limited, and any of the known methods such as immunoassays, high-performance liquid chromatography, and mass spectrometry can be used. In particular, it is preferable to measure canine TARC by an immunoassay using an antibody that specifically binds to the TARC. Examples of the immunoassay include a solid-phase immunoassay (e.g., RIA, EIA, FIA, CLIA), dot blotting, latex agglutination-turbidimetric immunoassay (LA), and immunochromatography. Among them, an ELISA (Enzyme-Linked ImmunoSorbent Assay) method, a type of EIA (Enzyme Immunoassay) method, is preferred to use from the viewpoint of quantitativity.

In the ELISA method, a sample is added to a microwell plate on which an anti-TARC antibody is immobilized and subjected to an antigen-antibody reaction; an enzyme-labeled anti-TARC antibody is further added, and an antigen-antibody reaction is performed; after washing, the complex is reacted with an enzyme substrate for coloring, and absorbance is measured to detect TARC in the sample; and the TARC concentration in the sample can be calculated from the measured values. Alternatively, fluorescence may also be measured after the antigen-antibody reaction by using a fluorescence-labeled anti-TARC antibody. The antigen-antibody reaction can be performed at 4°C to 45°C, more preferably 20°C to 40°C, and still more preferably 25°C to 38°C, and the reaction time is 10 minutes to 18 hours, more preferably 10 minutes to 1 hour, and still more preferably 30 minutes to 1 hour.

The ELISA method is not particularly limited, but is preferably a quantitative method. It is possible to adopt, as the quantitative method, a method in which a standard solution comprising canine TARC of known concentration is measured simultaneously with the sample, and the concentration of canine TARC in the sample is calculated based on a calibration curve prepared from the measured values of the standard solution.

The anti-TARC antibody used in an immunoassay may be any antibody that can bind to TARC. The anti-TARC antibody may be a monoclonal or polyclonal antibody, and functional fragments, such as Fab, Fab', and F(ab')₂ of a monoclonal antibody, that have activity of binding to TARC may also be used. The antibody is preferably immunoglobulin G (IgG). The animal from which the antibody is derived is not particularly limited, but it is preferable to use animals other than dogs. For example, the animal may be any of mice, rats, rabbits, goats, cows, pigs, sheep, cats, monkeys, camels, alpacas, birds, and fish. Preferably, the organism from which the antibody is derived is a mouse. Note that as used herein, the term "antibody" does not necessarily mean an intact antibody, but also includes binding fragments such as Fab, Fab', and F(ab')₂. Alternatively, the antibody may be a single-domain antibody. The single-domain antibody, also called a single-chain antibody or Nanobody(R), is an antibody consisting of the variable region of an antibody (immunoglobulin) composed only of heavy chains. VHH, which is found in camelid mammals, and VNAR, which is found in sharks, are known.

For example, the anti-TARC monoclonal antibody described in Patent Literature 1 may be suitably used as an anti-TARC antibody. The anti-TARC monoclonal antibody described in Patent Literature 1 is specifically an anti-canine TARC monoclonal antibody that binds to canine TARC or a functional fragment thereof that binds to canine TARC including a heavy chain variable region consisting of the amino acid sequence represented by SEQ ID NO: 2 and a light chain variable region consisting of the amino acid sequence represented by SEQ ID NO: 3.

### 1-3 Step b)

Step b) of the method of this embodiment is a step of determining that treatment for atopic dermatitis is useful when the level of TARC measured in step a) is equal to or greater than a threshold value, and determining that treatment for a different disease is useful when the level of TARC is less than the threshold value.

In the method of this embodiment, the setting of "threshold value" is not particularly limited and may be set in advance, or it may be set each time based on the measured values of the negative controls or standards by simultaneously measuring the negative controls or standards when the sample is measured. For example, a sample(s) collected from a healthy dog(s) or dog(s) affected by the different diseases mentioned above may be measured simultaneously as the negative controls. Here, healthy dogs refer to those not affected by dermatitis. It has been known that usually, the TARC concentration in the blood of dogs with atopic dermatitis is significantly higher than that in healthy dogs (e.g., Patent Literature 2). Thus, for example, the threshold value may be set to 2, 5, or 10 times the concentration in blood samples of the negative controls, especially healthy dogs. In this embodiment, the TARC value in a sample is also referred to as a TARC concentration or TARC level.

In the case of setting the threshold value in advance, for example, the level of TARC in the samples of healthy dogs or dogs affected by the different diseases mentioned above may be measured in advance, and the threshold value may be determined based on the measured values.

The threshold value may be determined, for example, by ROC (receiver operating characteristic curve) analysis. The decision accuracy (sensitivity and specificity) of the method of the present invention can also be determined by ROC analysis. The ROC analysis is a analysis method which is performed by measuring TARC levels in samples collected from a plurality of dogs suffering from canine atopic dermatitis and samples collected from a plurality of dogs suffering from the different diseases mentioned above, and calculating the sensitivity and specificity at each threshold value, and drawing a graph (curve) by plotting the results on the coordinates with the horizontal axis being "1 - specificity" and the vertical axis being "sensitivity", and the threshold value when the curve is closest to the upper left corner of the graph is adopted as the effective threshold value.

For example, the threshold value for TARC concentration in serum may be set at 500 to 1000 pg/mL. This threshold value is preferably the value measured by ELISA.

If the TARC concentration in a blood sample is equal to or greater than the threshold value, the test dog can be determined to be suffering from atopic dermatitis. On the other hand, if the TARC concentration in a blood sample is less than the threshold value despite the presence of an atopic dermatitis-like skin symptom, the test dog can be determined to be suffering from any of the different diseases mentioned above. Based on these results, one can determine that treatment for atopic dermatitis is useful for the former test dog while treatment for a different disease is useful for the latter test dog. Clinicians can use the results as an aid in making a decision about the treatment strategy for a dog with an atopic dermatitis-like skin symptom.

### 2. Second Embodiment - Reagent for assisting in determining treatment strategy

The reagent of the second embodiment of the present invention is a reagent for assisting in determining a treatment strategy for a canine subject with an atopic dermatitis-like skin symptom, wherein the reagent has a function to measure a level of TARC comprised in a blood sample collected from the subject, and the treatment strategy determination comprises determining that treatment for atopic dermatitis is useful when the level of TARC is equal to or greater than a threshold value, and determining that treatment for a different disease is useful when the level of TARC is less than the threshold value.

More specifically, the reagent, and the like of this embodiment are a reagent, and the like, used for the method of the first embodiment. The definitions of terms in this embodiment are the same as those in the first embodiment, unless otherwise stated.

The "reagent" herein may consist of a single composition or may be a combination of two or more compositions (e.g., a reagent set). Alternatively, the reagent may include one or more compositions in combination with a device (e.g., a kit). In other words, the term "reagent" herein includes a reagent set and/or a kit. For example, in the case of a reagent for measuring the level of TARC by ELISA, provided are a microwell plate on which an anti-TARC antibody is immobilized, a solution comprising a labeled anti-TARC antibody, a standard solution, a diluent, a washing solution, and the like. An instruction for use, and the like may be optionally provided.

The reagent of this embodiment involves a function to measure a level of TARC comprised in a blood sample. The function to measure a level of TARC in a blood sample is not particularly limited, and can be a function that enables known measuring methods such as an immunoassay, high-performance liquid chromatography, and mass spectrometry. In particular, it is preferable to provide an antibody that specifically binds to canine TARC as a function enabling the immunoassay. The immunoassay is not particularly limited, and from the viewpoint of quantitativity, it is preferable to use an ELISA method.

### 3. Third and Fourth Embodiments - Method for assisting in differential diagnosis and reagent for assisting in differential diagnosis

The third embodiment of the present invention is a method for assisting in a differential diagnosis of atopic dermatitis in a canine subject, comprising the following steps of:
a) measuring a level of TARC comprised in a blood sample collected from the subject; and
b) determining that the subject suffers from atopic dermatitis when the level of TARC is equal to or greater than a threshold value, and determining that the subject suffers from a different disease when the level of TARC is less than the threshold value.

The method of this embodiment allows for a differential diagnosis of whether a dog with a skin symptom is suffering from atopic dermatitis or a different disease by blood test. The configuration of the method of this embodiment, definitions of each term, and the like, are the same as those of the first embodiment, unless otherwise specified.

The fourth embodiment of the present invention is a reagent for assisting in a differential diagnosis of atopic dermatitis in a canine subject, wherein the reagent has a function to measure a level of TARC comprised in a blood sample collected from the subject, and the differential diagnosis comprises determining that the subject suffers from atopic dermatitis when the level of TARC is equal to or greater than a threshold value, and determining that the subject suffers from a different disease when the level of TARC is less than the threshold value.

The reagent of this embodiment is a reagent that can be used for a differential diagnosis of whether a dog with a skin symptom is suffering from atopic dermatitis or a different disease by blood test. More specifically, the reagent of this embodiment is a reagent used for the method of the third embodiment. The configuration of the reagent of this embodiment, definitions of each term, and the like are the same as those of the first and second embodiments, unless otherwise specified.

### Examples

The present invention is described in detail with reference to Examples below. The present invention, however, is not limited to these Examples.

Blood samples were collected from 30 dogs (10 cases in the atopic dermatitis (cAD) group, 10 cases in the scabies group, and 10 cases in the healthy group), and the level of TARC in each serum was measured. An anti-TARC monoclonal antibody CT-1 (see Patent Literature 1) was immobilized on a 96-well microplate by using 0.05 mol/L sodium carbonate buffer (pH 9.6). After washing, the plate was blocked for 2 hours at 37°C using Block Ace(R) (manufactured by MEGMILK SNOW BRAND Co., Ltd.). After the plate was washed, each serum sample diluted with diluent comprising TARC-negative canine serum was dispensed into wells and allowed to react for 1 hour at 37°C. After the plate was washed, a rabbit anti-canine TARC polyclonal antibody was added as a primary antibody and reacted at 37°C for 1 hour. The plate was then washed, and a horseradish peroxidase (HRP)-conjugated anti-rabbit IgG antibody was added as a secondary antibody and reacted at 37°C for 1 hour. After adding 3,3',5,5'-tetramethylbenzidine (TMB) as a substrate, coloring was allowed to develop at 37°C for 20 min, and the coloring was stopped by adding 0.5 mol/L sulfuric acid solution. The absorbance of each well was measured at 450 nm with a plate reader. Apart from the above serum samples, the same measurements were also performed for several recombinant canine TARC solutions of known concentrations, and a calibration curve was drawn based on the known concentrations and the absorbance values obtained. The level of TARC in each blood sample was calculated based on the calibration curve.

The amount of allergen-specific IgE was measured for the same serum samples. The recombinant tick antigen Derf2 was diluted with 0.05 mol/L sodium carbonate buffer (pH 9.6) and was immobilized on a 96-well microplate. After the plate was washed, a bovine serum albumin-comprising buffer solution was added for blocking. After the plate was washed, each serum sample diluted with a bovine serum albumin-comprising buffer solution was dispensed into wells and allowed to react for 1 hour at 37°C. After the plate was washed, a horseradish peroxidase (HRP)-conjugated anti-canine IgE antibody was added, and was reacted at 37°C for 1 hour. After adding 3,3',5,5'-tetramethylbenzidine (TMB) as a substrate, coloring was allowed to develop at 37°C for 30 min, and the coloring was stopped by adding 0.5 mol/L sulfuric acid solution. The absorbance of each well was measured at 450 nm with a microplate reader.

Table 1 shows the distribution of TARC levels in serum samples from each group. For each group, Mann Whittney test was performed to test a significant difference between the groups. p = 0.06 between the cAD group and the healthy group, and p = 0.03 between the cAD group and the scabies group. Between the cAD group and the scabies group, the cAD group exhibited significantly higher TARC levels. Table 2 shows the distribution of the amounts of IgE in serum samples from each group. Table 2 shows that there is no significant difference in the amount of IgE between each group.

**[Table 1]**

| | cAD | Scabies | Healthy |
|---|---|---|---|
| Number of cases | 10 | 10 | 10 |
| Minimum (ng/mL) | 0.05000 | 0.05000 | 0.05000 |
| 25% percentile (ng/mL) | 2.535 | 1.120 | 0.5008 |
| Median (ng/mL) | 4.931 | 2.445 | 2.209 |
| 75% percentile (ng/mL) | 10.09 | 3.205 | 3.734 |
| Maximum (ng/mL) | 16.99 | 8.109 | 9.725 |
| Mean (ng/mL) | 6.596 | 2.595 | 2.745 |
| Standard deviation | 5.721 | 2.274 | 2.821 |
| Standard deviation of mean | 1.809 | 0.7192 | 0.8922 |

**[Table 2]**

| | cAD | Scabies | Healthy |
|---|---|---|---|
| Number of cases | 10 | 10 | 10 |
| Minimum (IU/mL) | 0.05700 | 0.1140 | 0.06900 |
| 25% percentile (ng/mL) | 0.08850 | 0.3093 | 0.1278 |
| Median (IU/mL) | 0.1900 | 0.4925 | 0.2140 |
| 75% percentile (IU/mL) | 0.5053 | 0.7140 | 0.5713 |
| Maximum (IU/mL) | 0.8880 | 0.7750 | 0.8550 |
| Mean (IU/mL) | 0.3193 | 0.5006 | 0.3434 |
| Standard deviation | 0.2737 | 0.2238 | 0.2756 |
| Standard deviation of mean | 0.08655 | 0.07076 | 0.08716 |

Table 3 shows the amino acid sequences represented by SEQ ID NOs: 1 to 3 in the present disclosure.

**[Table 3]**

| SEQ ID NO: | Description | Amino acid sequence |
|---|---|---|
| 1 | Dog TARC | MIPLKMLLLVTLLLGASLQVTHAARGTNVGRECCLEYF KGAIPISRLTRWYKTSGECPKDAIVFVTVQGKSICSDPK DKRVKKAVRYLQRTWKGGPQES |
| 2 | Antibody heavy chain variable region | MGKCAAMGRLTSSFLLLIVPAYVLSQVTLKESGPGILQP SQTLSLTCSFSGFSLNTSGMGVGWIRQPSGKGLEWLAHI WWDDDKRYNPALKSRLTISKDTFNNQVFLNIASVDTAD TATYYCARIYFGSSLAWFAYWGQGTLVTVSA |
| 3 | Antibody light chain variable region | MGTQDFLYQVLRMRCLAEFLGLLVLWIPGAIGDIVMTQ ATSSVFVTPGDSVSISCRSSKSLLHSNGNTYLYWFLQRP GQSPQLLIYRMSNLASGVPDRFSGSGSGTAFTLRISRVE AEDVGVYYCMQHLENPTFGAGTKLELKR |

All the publications, patents, and patent applications cited herein are incorporated herein by reference in the entirety.

## Claims

1. A method for assisting in determining a treatment strategy for a canine subject with an atopic dermatitis-like skin symptom, comprising the steps of:
a) measuring a level of TARC comprised in a blood sample collected from the subject; and
b) determining that treatment for atopic dermatitis is useful when the level of TARC is equal to or greater than a threshold value, and determining that treatment for a different disease is useful when the level of TARC is less than the threshold value.

2. The method according to claim 1, wherein in the step b), the different disease is at least one disease selected from the group consisting of scabies, acne mites, flea parasites, tick parasites, lice parasites, bacterial infections, fungal infections (including yeast infections), immune-mediated diseases (including psoriasis and food allergy), stress-induced scratching, mastocytoma, and cutaneous lymphomas.

3. The method according to claim 2, wherein the different disease is an infection.

4. The method according to claim 1, wherein in the step a), the level of TARC is measured using an antibody that specifically binds to canine TARC.

5. A reagent for assisting in determining a treatment strategy for a canine subject with an atopic dermatitis-like skin symptom, wherein
the reagent has a function to measure a level of TARC comprised in a blood sample collected from the subject, and
the treatment strategy determination comprises determining that treatment for atopic dermatitis is useful when the level of TARC is equal to or greater than a threshold value, and determining that treatment for a different disease is useful when the level of TARC is less than the threshold value.

6. The reagent according to claim 5, wherein the different disease is at least one disease selected from the group consisting of scabies, acne mites, flea parasites, tick parasites, lice parasites, bacterial infections, fungal infections (including yeast infections), immune-mediated diseases (including psoriasis and food allergy), stress-induced scratching, mastocytoma, and cutaneous lymphomas.

7. The reagent according to claim 6, wherein the different disease is scabies.

8. The reagent according to claim 5, comprising an antibody that specifically binds to canine TARC.
